# EUROPEAN PATENT SPECIFICATION

(11) **EP 4 608 253 B1**
(45) Date of publication and mention of the grant of the patent: **17.06.2026**
(21) Application number: 23792954.2
(22) Date of filing: 18.10.2023
(51) Int. Cl.: A61B 5/021, A61B 5/022

(54) **CUFF AND SYSTEM FOR HEMODYNAMIC MONITORING**
MANSCHETTE UND SYSTEM ZUR HÄMODYNAMISCHEN ÜBERWACHUNG
BRASSARD ET SYSTÈME DE SURVEILLANCE HÉMODYNAMIQUE

(30) Priority: 26.10.2022 EP 22203777
(43) Date of publication of application: 03.09.2025
(73) Proprietor: Koninklijke Philips N.V., 5656 AE Eindhoven (NL)
(72) Inventor: DAVIDOIU, Valentina-Geta, 5656 AG Eindhoven (NL); HILGERS, Achim Rudolf, 5656 AG Eindhoven (NL); MENA BENITO, Maria Estrella, 5656 AG Eindhoven (NL); SCHMITT, Lars, 5656 AG Eindhoven (NL); PELSSERS, Eduard Gerard Marie, 5656 AG Eindhoven (NL); WIJSHOFF, Ralph Wilhelm Christianus Gemma Rosa, 5656 AG Eindhoven (NL)
(74) Representative: Philips Intellectual Property & Standards
(86) International application number: PCT/EP2023/078936
(87) International publication number: WO 2024/088842

(56) References cited:
- US-A1- 2018 353 091
- US-A1- 2019 387 987

## Description

### FIELD OF THE INVENTION

The present invention relates to a cuff and a system for hemodynamic monitoring.

### BACKGROUND OF THE INVENTION

Arterial blood pressure (BP), as well as heart rate, respiratory rate, oxygen saturation, and body temperature, are vital signs reflecting the cardiovascular status of a human. In order to prevent hypoperfusion and to guide fluid administration, blood pressure (BP) and cardiac output (CO) need to be continuously monitored. In other words, the availability of hemodynamic monitoring ensures optimal tissue perfusion and oxygen delivery while maintaining adequate the mean arterial blood pressure of the patient. BP can either be obtained invasively via an arterial blood-pressure catheter (ABP) or non-invasively via a BP air cuff (NIBP).

The invasive BP monitoring is usually indicated only in the case of high-risk patients or in complex surgical procedures. The major advantage of an ABP is that it is a direct pressure measurement and offers the possibility of blood sampling for physiological monitoring and hemodynamics monitoring but also medication titration. Via an ABP a continuous beat-to-beat blood pressure measurement is acquired. For this reason, it is used in operating rooms (OR), intensive care units (ICU) or emergency departments (ED) and is regarded as the "gold standard" in critically ill patients. Nevertheless, ABP is only used in high-risk patients because it is associated with adverse effects including infections, thrombosis, bleeding, distal ischemia and patient immobility. It is also well known that in clinical practice a clear trend is to minimize the use of ABP and to switch from ABP to NIBP as early as possible in order to avoid complications and infections.

In this context, continuous advanced hemodynamic monitoring is available only to few patients. Moreover, the large majority of the patients are monitored using traditional non-invasive technologies (e.g. NIBP, SpO2, ECG, respiration rate). Recently, it was proven that short periods of hypotension may be unrecognized when using intermittent BP monitoring. In addition, large datasets have demonstrated that even short periods of low BP (or their cumulative duration) may have a detrimental impact on the development of postoperative outcome including increased risk of acute kidney injury, myocardial injury development or even death. On the other hand, the NIBP technology supports only BP measurements and does not offer any additional hemodynamic parameter (e.g. cardiac output, stroke volume, etc.).

US 2015/359446 A1 discloses a blood pressure measuring system configured to surround a patient's body part, comprising pressurization means for applying pressure to the body part, and comprising a kinking-proof shell, wherein the kinking-proof shell is arranged so as to be located between the pressurization means and the body part, when the blood pressure measuring system surrounds the body part. In an embodiment, the kinking-proof shell may comprise a plurality of individual shell elements or shell sections, which may exhibit, e.g., differing thicknesses and/or differing moduli of elasticity. Such a blood pressure measuring system is also referred to as Advanced Monitoring Cuff (AMC).

US 2018/353091 A1 discloses a finger cuff that is connectable to a patient's finger to aid in measuring the patient's blood pressure. The finger cuff may comprise: a shell, a bladder, and a clamping mechanism. The shell may have a finger cavity. The finger cavity of the shell may be placed under a patient's finger to receive the patient's finger. Further, the finger cavity may include a light emitting diode (LED)-photodiode (PD) pair. The bladder may include a pair of openings and the bladder may be mountable within the finger cavity such that the pair of openings surround the LED-PD pair, respectively. The clamping mechanism may be used to suitably clamp the patient's finger received in the finger cavity of the shell against the bladder mounted within the finger cavity of the shell.

US 2019/387987 A1 discloses a method to collect cardiovascular data relating to an individual user, in a system comprising an acoustic sensor configured to be coupled to the chest of the user, an arm band having an inflatable bladder configured to be placed around at a predefined position around a upper limb of the user, for example the left arm, a pneumatic unit with at least a pump and a pressure sensor, configured to inflate and deflate the inflatable bladder.

### SUMMARY OF THE INVENTION

It is an object of the present invention to provide a cuff and system for hemodynamic monitoring that are further improved with respect to one or more of measurement quality, flexibility of use and production costs.

In a first aspect of the present invention a cuff for hemodynamic monitoring is presented, comprising:
- a shell configured to enclose a subject's body part during hemodynamic monitoring, the shell comprising at least a first shell part and a second shell part having a higher rigidity than the first shell part, each shell part being configured for enclosing a different circumferential portion of the body part;
- a sensor unit arranged in or at an inner surface of at least one shell part and configured to measure hemodynamic signals;
- one or more closing elements arranged in or at at least one shell part and configured to hold the shell parts together when mounted at the body part; and
- an actuator configured to pressurize and/or compress at least one of the shell parts,

wherein the first shell part is compressible and/or pressurizable and the second shell part is rigid or semi-rigid, and
wherein i) the first shell part represents the actuator or integrates the actuator or ii) the actuator is arranged on an outer surface of the first shell part or the second shell part.

In a further aspect of the present invention a system for hemodynamic monitoring is presented comprising:
- a cuff as disclosed herein;
- an actuator controller connected to the actuator of the cuff and configured to control the actuator to press at least one of the shell parts of the cuff onto the body part; and
- a monitor connected to the sensor unit and configured to obtain a measurement signal from the sensor unit.

Preferred embodiments of the invention are defined in the dependent claims. It shall be understood that the claimed system has similar and/or identical preferred embodiments as the claimed cuff, in particular as defined in the dependent claims and as disclosed herein.

It has been recognized that the current AMC design, as e.g. disclosed in US 2015/359446 A1 may be further improved in one or more aspects. The current AMC design requires a proper cuff size in order to fit as good as possible to the geometry of body part on which it shall be used, e.g. the human upper arm, and to ensure that the body part is correctly compressed so that the brachial artery collapses. Further, depending on the body part, it may be hard to place the cuff properly, e.g. by sliding it over the hand up to the upper arm. Further, slip-stick artifacts may occur due to the rigid shell when two parts of the shell slip on each other.

The present invention is based on the idea to avoid some or all of these problems by using a "mixed" shell that comprises at least two shell parts having different rigidity, i.e., one of the shell parts is less rigid than the other shell part. The shell is thus at least partly semi-rigid or compressible. The cuff can be placed at the intended body part, preferably the upper arm, like a normal NIBP cuff, which makes it user friendly and ensures that the sensor unit (e.g. a sensor pad) is overlapping with the artery area and not with another non-intended part of the body, e.g. a side of the arm. The sensor unit is not covered by a liner as in the known AMC design so that a substantial drop (e.g. of 40 %) in signal quality is avoided.

Further, the at least two shell parts of the shell do not slip on each other so that slip-stick artifacts do not occur, which ensures the best signal quality. The actuator, e.g. an air pump, benefits as well from the design of the shell having two shell parts, wherein one of shell parts is softer than the other shell part (and the one softer shell part is also softer than the shell in the known AMC design).

According to an embodiment the first shell part is compressible, in particular by more than 20 % or by more than 30 %, and the second shell part is rigid or semi-rigid, in particular not compressible or less compressible than the first shell (e.g. only flexible / bendable by a small amount). The amount of compression may be chosen in a reasonable range or at a reasonable value, e.g., depending on one or more of the different parameters such as material, size and application (at which body part, for which kinds of patients, e.g. children, adults, etc.) of the cuff.

The first shell part is preferably made of material, such as polymer and/or foam and/or other shape memory material, that can adapt to the shape of the body part when compressed. The first shell part thus optimally adapts to the shape of the body part, e.g. the upper arm, when used and thus optimally pressurizes the body part. When pressure is released, the first shell part returns completely or largely to the original state so that the cuff can be easily removed from the body part and later attached again to the body part of the patient.

In an embodiment the first shell part represents the actuator or integrates the actuator. For instance, the first shell part can be directly inflated or pressurized so that no additional elements are required for this process.

In an alternative embodiment the actuator is arranged on an outer surface of the first shell part or the second shell part. For instance, an inflatable bladder may be arranged on the outer surface of the first shell part, which may be connected via an air hose to an air pump to inflate the bladder and thus to pressurize or compress the first shell part. Preferably, the actuator is arranged on an outer surface of the shell part in which the sensor unit is arranged, which preferably is the more rigid second shell part.

In still another embodiment the cuff further comprises a fixation that is fixed at an outer surface of the first shell part or the second shell part and coupled to the actuator, wherein the actuator is configured to be removably fixed to an outer surface of the first shell part or the second shell part when the cuff is mounted onto the body part. The actuator can thus e.g. be fixed to one of the shell parts (e.g. the first shell part), when the cuff shall be used, and can be removed from this shell part after use to depressurize this shell part. Via the fixation the actuator may be fixedly connected the outer surface of the first or second shell part so that it cannot get lost or removed from the cuff. Generally, however, via this fixation the first or second shell part is not pressurized or compressed when the actuator is fixed to the respective other shell part during actual use of the cuff.

The shell may have a conical shape and/or may form a concentrical shell without overlapping portions in circumferential direction. This enables easy mounting, e.g. on the upper arm, and avoids slip-stick artifacts during use when the cuff is slowly pressurized during the hemodynamic measurement.

Preferably, the first shell part or the second shell part is configured to be arranged at an inner part of the upper arm. Preferably, the shell part in which the sensor unit is arranged is placed above the inner part of the upper arm. This ensures that it is located over the brachial artery at the inner side of the arm, where it would be best and where it is covered by the more rigid shell part.

The shell parts may be configured as half shells each being configured to enclose the body part in a circumferential area of substantially 180°. This allows an easier manufacture and assembly of the shell as well as an easy handling and mounting of the cuff, e.g. to an upper arm. Further, such a configuration may allow to reuse the rigid shell that can be easily disinfected / sterilized, i.e., this configuration may be more sustainable.

In a preferred embodiment the one or more closing elements comprise magnetic elements arranged at one or more opposing end surfaces of the shell parts. The two shell parts can thus be easily assembled when the cuff is mounted e.g. to the upper arm and will then be held together by the magnetic elements. For removal of the cuff the two shell parts can then be simply separated and removed from the upper arm separately. Thus, both for mounting and removal of the cuff it is not required to slide the cuff over the complete arm as is needed with the AMC design disclosed e.g. in US 2015/359446 A1, which is thus more comfortable for a patient.

The sensor unit is preferably integrated into the inner surface or arranged onto the inner surface of the first shell part or the second shell part. It can thus be easily placed at the right position to measure the BP at the brachial artery. Further, the sensor unit is generally not covered by any textile as in the known AMC design, which further improves the accuracy and reliability of hemodynamic measurements (a BP measurement) made by use of the cuff according to the present invention.

In a practical implementation the sensor unit comprises a sensor pad filled with a fluid, a pressure transducer configured to convert a fluid pressure into an electrical measurement signal, and a fluid connection between the sensor pad and the pressure transducer.

The actuator may comprise, in an implementation, an inflatable bladder and an air connection connected at one end to the inflatable bladder. An external air pump may thus be used to inflate the bladder during use.

The disclosed system for hemodynamic monitoring generally comprises the cuff as described above, an actuator controller connected to the actuator of the cuff and configured to control the actuator to press at least one of the shell part of the cuff onto the body part, and a monitor (e.g. a conventional patient monitor or a separate monitor) connected to the sensor unit and configured to obtain a measurement signal from the sensor unit. The actuator controller may comprise an air pump to inflate an inflatable bladder of the actuator through an air hose. The actuator controller may be part of the monitor.

According to a further aspect of the present invention a cuff for hemodynamic monitoring is presented, comprising:
- a shell configured to enclose a subject's body part during hemodynamic monitoring, the shell comprising at least a first shell part and a second shell part having a higher rigidity than the first shell part, each shell part being configured for enclosing a different circumferential portion of the body part;
- a sensor unit arranged in or at an inner surface of at least one shell part and configured to measure hemodynamic signals;
- one or more closing elements arranged in or at at least one shell part and configured to hold the shell parts together when mounted at the body part; and
- an actuator configured to pressurize and/or compress at least one of the shell parts.

### BRIEF DESCRIPTION OF THE DRAWINGS

These and other aspects of the invention will be apparent from and elucidated with reference to the embodiment(s) described hereinafter. In the following drawings
Fig. 1A shows an embodiment of a known cuff in a deflated state.
Fig. 1B shows an embodiment of the known cuff shown in Fig. 1A in an inflated state.
Fig. 2 shows a diagram of a pressure curve over time for the known cuff shown in Figs. 1A and 1B.
Fig. 3A shows a cross-sectional view of a schematic diagram of a first embodiment of a cuff according to the present invention in an uncompressed / deflated state.
Fig. 3B shows a cross-sectional view of the cuff shown in Fig. 3A in a compressed / inflated state.
Fig. 4 shows a schematic diagram of an embodiment of a system according to the present invention.
Fig. 5 shows a schematic diagram of a second embodiment of a cuff according to the present invention.
Fig. 6 shows a schematic diagram of an embodiment of a sensor unit according to the present invention.
Fig. 7 shows a schematic diagram of a third embodiment of a cuff according to the present invention.
Fig. 8A shows a schematic diagram of a fourth embodiment of a cuff according to the present invention. in an uncompressed / deflated state.
Fig. 8B shows a cross-sectional view of the cuff shown in Fig. 8A in a compressed / inflated state.
Fig. 9 shows a schematic diagram of a fifth embodiment of a cuff according to the present invention.
Fig. 10 shows a schematic diagram of a sixth embodiment of a cuff according to the present invention.
Fig. 11 shows a schematic diagram of a seventh embodiment of a cuff according to the present invention.
Fig. 12 shows a schematic diagram of an eighth embodiment of a cuff according to the present invention.

### DETAILED DESCRIPTION OF EMBODIMENTS

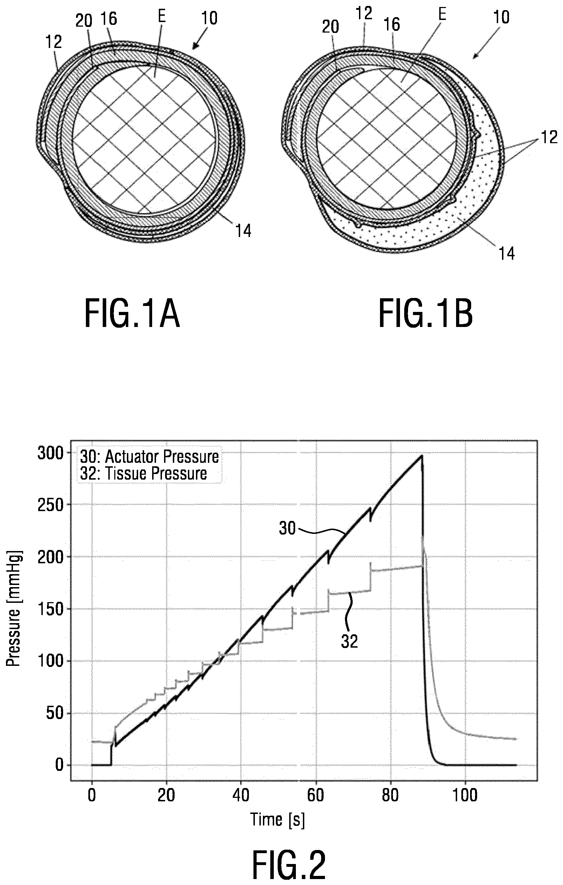

Fig. 1 shows an embodiment of a known cuff 10, as e.g. disclosed in US 2015/359446 A1, in a deflated state (Fig. 1A) and in an inflated state (Fig. 1B). The cuff 10 (also called Advanced Monitoring Cuff (AMC) herein) uses AMC technology and a kinking-proof shell 20 that is arranged between the pressurization means, comprising a pressure actuator 12 with an air/fluid bag 14, and the body part E (e.g. a subject's upper arm). The kinking-proof shell 20 is arranged (sandwiched) between the pressure actuator 12 with the air/fluid bag 14 and a flexible element 16.

The kinking-proof shell 20 is preferably made from plastic material, such as polyethylene. The thickness of the kinking-proof shell 20 is chosen so that the kinking-proof shell 20 does not buckle when pressure is applied to the air/fluid bag 14, while at the same time the kinking-proof shell 20 is flexible enough to allow for a certain deformation of the kinking-proof shell 20. That is, when pressure is applied to the air/fluid bag 14, overlapping edge regions of the kinking-proof shell 20 move or slide relatively to each other so as to reduce the diameter of the kinking- proof shell 20. However, the kinking-proof shell 20 thereby remains substantially ring-shaped. The kinking-proof shell 20 may comprise individually formed shell elements that are substantially in parallel to each other to better fit or adapt to the shape of the body part E.

The cuff 10 shown in Fig. 1 is a disposable cuff meant to be used for a maximum of 72 hours on critically ill patients in the operating room (OR) or intensive care unit (ICU) who are intubated and require fluid management. AMC is a non-invasive technology that requires to place a non-invasive cuff around the upper arm of a patient. Once the AMC is placed on the human upper arm it is connected to a patient monitor (PM) via an electrical cable and an air hose. A measurement is completed after 90 seconds, and the clinician receives on the PM several advanced hemodynamic parameters, wherein the most important ones are blood pressure (BP), cardiac output (CO) and stroke volume (SV).

In a practical implementation an AMC is a component of a hemodynamic monitoring system and comprises three main elements (also shown in Fig. 1):
(a) An inflatable bladder (also referred to as "actuator") which is attached to the air hose. When inflated, the actuator compresses the hard plastic conical shell round the patient's upper arm.
(b) A hard plastic conical shell, referred to herein as the "shell", which is surrounded and bound by the actuator and serves to compress the patient's arm when the actuator is inflated via the air hose of the PM. The interior of the shell, when assembled, is covered by a liner/textile to prevent pinching of the patient skin.
(c) A "sensor assembly" comprising a fluid-filled bag (referred to herein as the "sensor pad"), located on the interior surface of the rigid shell, which is connected by fluid-filled tubing to a disposable pressure transducer (DPT) mountable on the exterior of the AMC, which is connected by an electrical cable to the PM. When the actuator is inflated, the shell compresses the sensor pad against the patient's upper arm, and the tissue pressure of the patient's arm is hydraulically conducted to the DPT. The DPT translates the fluid pressure to an analog electrical signal which is conducted by means of the electrical cable to the monitor, where the brachial arterial pulsation (beat-to-beat blood flow) is analyzed.

The electrical cable attached to the DPT may contain a 1-wire authentication circuitry to identify the cuff size and other information stored therein. The actuator may connect to a standard NIBP air hose or an AMC hose assembly.

Several steps are needed to place an AMC cuff on a patient's arm. The AMC cannot be used as straightforward as an NIBP cuff in general. In particular, the clinician needs to measure the patient arm circumference upfront. Based on this measurement the correct AMC size is determined and the AMC is used then. Since humans are very different the AMC may be available in different cuff sizes to accommodate for different arm geometries, e.g. for small adults, adults and large adults. If the size of the AMC is wrongly assessed, this can end-up in a wrong measurement and faulty values of the BP, CO or SV. Therefore, the cuff size is playing an important role in order to ensure a correct AMC measurement. Another possible side effect of the wrong cuff size assessment may be that the cuff it is too large for the patient upper arm and during inflation the arm is not well compressed, and the brachial artery is not occluded. Even more general, the number of different sizes of AMC may be limited and may thus not be sufficient to cover all the human arm geometries.

Besides the correct cuff size, several other aspects of the current AMC design can affect and reduce the accuracy of the measurement. One of these elements are the mechanical artifacts created by the plastic rigid shell when the AMC is inflated, and the human upper arm is compressed. This artifact is known as "slip-stick" artifact and is shown in Fig. 2 that shows the actuator pressure 30 and the tissue pressure 32. Practically, a pressure force will be exercised on the shell during inflation, and this can result in a friction phenomenon between the two extremities of the plastic rigid shell that will slide against each other. As a result, the circumference of the shell suddenly changes and therefore causes a change in the cuff pressure, which does hence not increase steadily but shows multiple abrupt rises during inflation as shown in Fig. 2.

Another disadvantage of the rigid shell is that it requires a very high actuator air pressure to cope for mechanical losses while compressing the upper arm. In daily practice, the air pump that is normally available in the PM for the traditional NIBP measurement has a maximum pressure limit of 300 mmHg. For the AMC measurements this upper limit needs to be higher (up to 650 mmHg) in order to be able to compress the plastic shell, and finally to occlude the brachial artery. This type of very powerful pumps capable to meet this high-pressure requirement may have difficulties to provide low flow rates for low cuff pressure, which are needed to avoid that inflation rate is too high at low cuff pressure (around diastolic pressure). Hence, there may be conflicting requirements: high peak pressure and low flow rate at low pressure. The cuff presented according to the present invention can overcome this conflict.

Still another disadvantage of the rigid shell is that for comfort reasons it is covered with a liner or textile, e.g. a white interior cloth, to improve the sliding of the rigid shell on the skin and not to pinch the skin. This may lead to a substantial reduction of signal quality because the liner is covering the sensor pad area, which is hence not in direct contact with the human skin/arm.

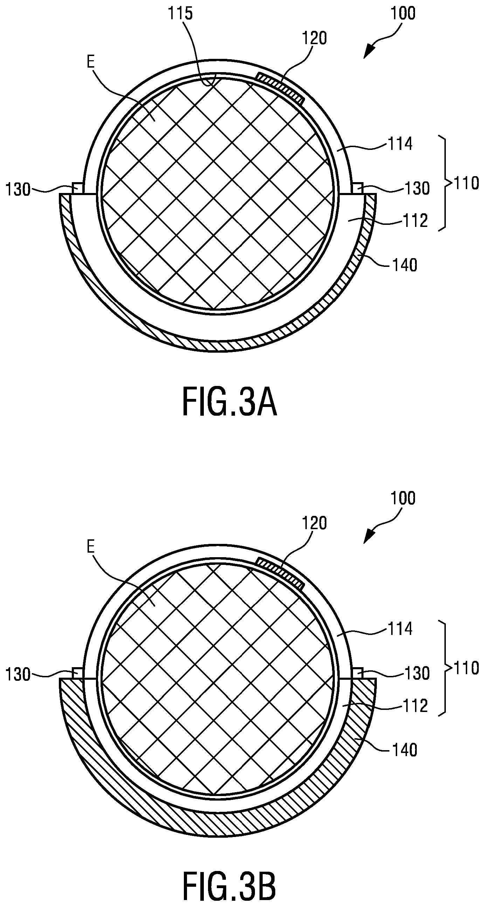

Fig. 3A shows a cross-sectional view of a schematic diagram of a first embodiment of a cuff 100 for (e.g. intermittent or continuous) hemodynamic monitoring according to the present invention (in uncompressed / deflated state). The cuff 100 comprises a shell 110 configured to enclose a subject's body part E (e.g. an upper arm) during hemodynamic monitoring. The shell 110 comprises at least a first shell part 112 and a second shell part 114 having a higher rigidity than the first shell part 112. Both shell parts 112, 114 are configured for enclosing a different circumferential portion of the body part. The cuff 100 further comprises a sensor unit 120 arranged in or at an inner surface 115 of at least one shell part, preferably of the second shell part 114, and configured to measure hemodynamic signals. One or more closing elements 130 (e.g. magnets, mechanical clamps, latches, Velcro fasteners, etc.) are arranged in or at at least one shell part, preferably at both shell parts 112, 114, which are configured to hold the shell parts 112, 114 together when mounted at the body part E. An actuator 140 is provided and configured to pressurize and/or compress at least the first shell part 112. In this embodiment, the actuator 140 is a separate element arranged around the outer surface of the first shell part 112.

Fig. 3B shows a cross-sectional view of the cuff 100 shown in Fig. 3A in a compressed / inflated state. As can be seen in Fig. 3B, only the first (semi-rigid or at least less rigid than the second shell part 114) shell part 112 is compressed and thus pressurizes the underlying tissue of the body part E so that the brachial artery collapses.

The cuff 100 shown in Fig. 3 may preferably be arranged such that the second shell part 114 is arranged at the inner part of the upper arm. Hence, the actuator 140 directly compresses the first shell part 112 at the outer side of the arm. However, this compression affects the second shell part 114 as well which is then "pulled" towards the brachial artery by increasing its volume and thus decreasing the encapsulated volume. Thus, in such an embodiment, the desired effect compressing the inner part of the arm so that the brachial artery collapses will be achieved.

The mixed shell 110 having shell parts of different rigidity as provided in the cuff according to the present invention, for which a number of different embodiments are disclosed herein, provides that it is not required to select the proper cuff size in order to fit as good as possible the geometry of the body part, as required in the known AMC to ensure that the arm is correctly compressed. Further, the cuff 100 according to the present invention is more user-friendly since the shell parts can preferably be separated to be mounted to the body part separately, whereafter they will be held together by the closing elements. It may thus not be required to slide the cuff over the body part (e.g. the complete arm), although this is generally possible as well (or may be required in certain embodiments). Embodiments of the cuff 100 may thus be more easily placed at the correct position so that the sensor unit 120 overlaps with or covers the artery area instead of any other undesired portion of the body part.

The two (or more) shell parts 112, 114 of the shell 110 are arranged and configured so that they do not slip on each other. Slip-stick artifacts as in the known cuff 10 can hence not occur, which ensures a better signal quality. The different rigidity of the shell parts, where one of them is softer and not fully rigid as the plastic shell in the known cuff 10 provides the further advantage that the air pump can be dimensioned smaller since a lower maximum pressure / air volume needs to be delivered. Finally, a textile liner does not cover the sensor unit 120 which avoids a substantial drop in signal quality.

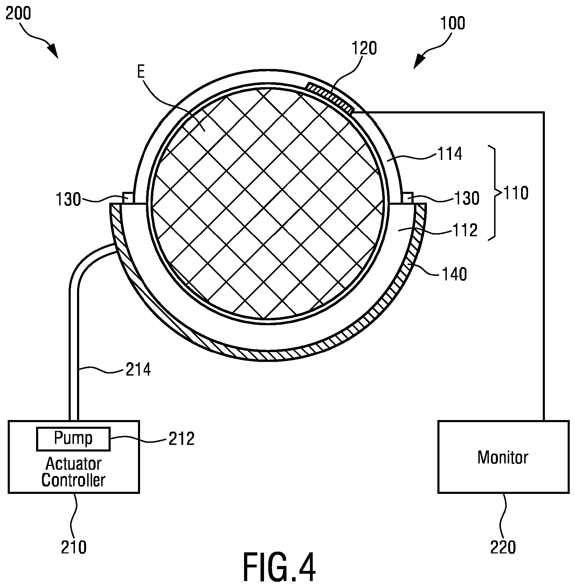

Fig. 4 shows a schematic diagram of an embodiment of a system 200 for hemodynamic monitoring according to the present invention. The system 200 comprises a cuff as according to the present invention as disclosed herein, e.g. a cuff 100 as shown in Fig. 3. The system 200 further comprises an actuator controller 210 that is connected to the actuator 140 of the cuff 100 and that is configured to control the actuator 140 to press the first shell part 112 of the cuff 100 onto the body part 100. Still further, the system 200 comprises a monitor 220 connected to the sensor unit 120 and configured to obtain a measurement signal from the sensor unit 120. The actuator controller 210 may comprise an air pump 212 and to inflate an inflatable bladder of the actuator 140 through an air hose 214. The actuator controller 210 may be a separate unit as shown in Fig. 4, but may be part of the monitor 220 in another embodiment.

According to an embodiment of the cuff according to the present invention, e.g. the cuff 100 shown in Fig. 3, the first shell part 112 may be semi-rigid or compressible. It may be made from a smart/responsive material, such as a shape memory foam or polymer, that adapts to the shape of the patient's body part, e.g. the upper arm, while it is being compressed. Once compressed, the shape memory foam is still hard to a certain extent, i.e. "rigid" in the sense that it is having a high-density index. Even if such a smart/responsive material may be very dense, it is less rigid than the plastic shell of the known cuff 10 and as the second shell part 114.

It shall be noted that in other embodiments first shell part 112 may be made from different materials, e.g. in the form of a layer structure. These layers may be horizontally (in circumferential direction) or vertically (in radial direction) positioned, e.g. resulting in a sandwich design with different rigidities.

From this semi-rigid aspect of the mixed design of the cuff 100, the air pump that is used to inflate the actuator according to an embodiment will benefit. The air pressure that is required to compress such a smart/responsive material is much lower than the air pressure needed to compress a full plastic sheet. Moreover, since such a smart/responsive material can adapt to the shape of the body part, the differences in the geometry of the body part, e.g. the thickness of the arm of different patients, is also addressed. In particular, such a smart/responsive material has the characteristic to get in touch with the human body part, e.g. human arm, faster, even at low inflation rates, which is an important aspect to support a decrease of the time interval needed to build up the required pressure and thus the moment when the actual compression of the body part starts. This time interval can be very long, even in traditional NIBP measurements, because a large air volume is needed to enter the cuff bladder so that the cuff gets in contact with the arm. This phenomenon is well known and seen in NIBP measurements when the cuff is loosely wrapped around the arm, so the distance from the cuff to the arm is larger compared with a situation when a cuff is very tightly wrapped around the arm.

Another benefit of the use of foam or polymer is that it is elastic. During the compression of the body part, it will follow the geometry of the body part and will increase the contact area and therefore allow for a pressure distribution which is more uniform. This uniform pressure distribution is also beneficial for the patient comfort because the pressure is distributed over a larger fraction of the body part in a better way. In contrast to an inner liner as used in the known cuff 10, the polymer has also the advantage that it will not fold during inflation.

Since the first and second shell parts 112 and 114 do not slide on each other, the slip-stick artifact is not present so that a better signal quality can be ensured. Moreover, the edges of the second (more rigid) shell part 114 can be covered with a protection, e.g. a cushioning, at the areas where it contacts or is adjacent to the first shell part 112 to avoid that a rigid edge of the second shell part injures or punches the patient's skin.

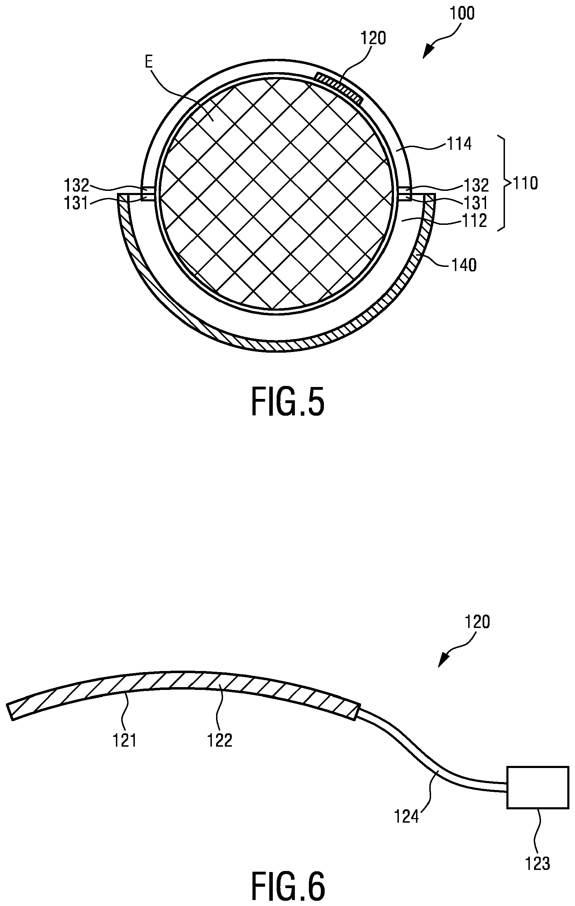

Fig. 5 shows a schematic diagram of a second embodiment of a cuff 100 according to the present invention (in uncompressed / deflated state). In this embodiment a magnetic closure of the shell is provided. In particular, the design of the cuff 100 shown in Fig. 5 is the same as in the first embodiment shown in Fig. 3, but the shell parts 112, 114 are held together by closing elements in the form of magnets 131, 132. In particular, first magnets 131 are arranged in (or on) the front surfaces (or edges) of the first shell part 112 and second magnets 132 (of opposite polarity) are arranged in (or on) the front surfaces (or edges) of the second shell part 114 facing the front surfaces (or edges) of the first shell part 112. Hence, the first and second shell parts 112, 114 may be easily and removably put together so that they are held together by the magnets 131, 132.

To mount this second embodiment of the cuff 100 to the patient's arm, the user (e.g. a clinician) will assess where the artery is located and will place the more rigid second shell part 114 such that the sensor unit 120 is placed over the artery. After the correct positioning of the sensor unit 120, the first shell part 112 is attached. The mounting of the cuff is thus easy, and also in the second embodiment the two shell parts 112, 114 are not sliding over each other. In a next step the actuator 140 (e.g. air cuff) is placed (wrapped) around the complete shell 110 like a normal NIBP cuff and may be held / closed e.g. with a Velcro mechanism.

According to a slightly modified embodiment magnets (or other closing elements) are only used at one side (e.g. in Fig. 5 on the left-hand side) of the shell parts, while the other side (in the example on the right-hand side) allows for a flexible opening/closing, i.e., on the other side the two shell parts are connected by a hinge or joint or other element that allows such an opening and closing. This allows for applying the shell 110 from a lateral side of a patient by first softly pulling the open ends (where the magnets are) apart, enclosing the upper arm, and finally closing the ends with the magnets. After that, the actuator 140 (air cuff) can be wrapped around the shell like a standard NIBP cuff. Moreover, in order to prevent any skin injury, the closing element (e.g. magnets) may be flat strips, which are in the same plane as the skin so that no rigid elements can hurt the patient during cuff inflation or deflation.

All other benefits described above for the first embodiment are given for the second (and further) embodiment(s) as well, while the placement of the cuff is different and more user-friendly.

As explained above, the placement of the sensor unit 120 on the artery is important to ensure high signal quality because this is the location of the signal that shall be measured and where the highest pulse pressure (signal) amplitude does appear. The sensor unit 120, e.g. a sensor pad, may be is glued onto the inner surface of the shell, preferably the second shell part 114 (or, alternatively the first shell part 112, as will be explained below with reference to other embodiments). Since this may cause different artifacts on the tissue and actuator pressure signals, the sensor unit 120 is preferably integrated into the first or second shell part . Preferably, the sensor unit 120 is designed in the form of a sensor pad and is curved so that it lays alongside the arm. A liner that covers the shell in the known cuff 10 is preferably not present, at least not above the sensor unit 120 but preferably not on the inner surface of the shell at all.

In an embodiment, the sensor unit 120 comprises a silicon bag for the sensor pad in direct contact with the patient arm so that the quality of the signal is not affected by any textile interference. Further, the sensor pad may slightly protrude from the inner surface of the shell to contribute to an increase of the signal quality as well. In an embodiment as schematically shown in Fig. 6, the sensor unit 120 may comprise a sensor pad 121 filled with a fluid 122, a pressure transducer 123 configured to convert a fluid pressure into an electrical measurement signal, and a fluid connection 124 between the sensor pad and the pressure transducer.

In another embodiment (not shown) the configuration is opposite to the configuration shown in Figs. 3 and 5. In particular, the first and second shell parts are exchanged, i.e., the first shell part 112 carries the sensor unit 120 and is arranged at the inner part of the arm and the second shell part 114 is arranged at the outer part of the arm. This configuration may be applied to all other embodiments as well.

In yet another embodiment the shell may be composed of multiple (more than two) shell part or segments. For instance, rigid parts and semi-rigid / compressible parts may be alternating in circumferential direction. For instance, the shell may comprise four (or six) segments having two (or three) rigid and two (or three) semi-rigid / compressible segments, where a semi-rigid / compressible segment is arranged between two rigid segments.

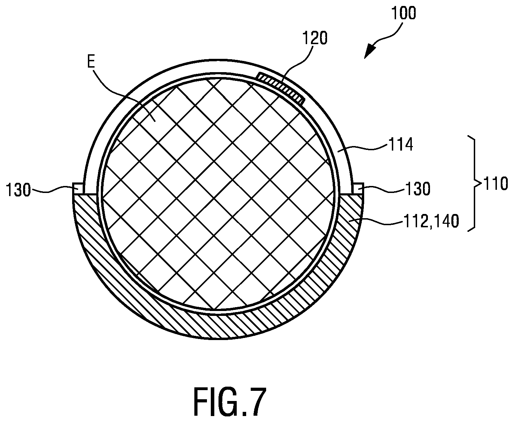

Fig. 7 shows a schematic diagram of a third embodiment of a cuff 100 according to the present invention (in uncompressed / deflated state). In this embodiment, the first shell part 112 represents the actuator 140 or integrates the actuator. For instance, the first shell part may be made of or include an inflatable bladder which can directly be inflated and deflated. This has the advantage that instead of two elements a single element fulfills the functions of the first shell part and the actuator.

It shall be noted in this context that. in this embodiment or in any other embodiment, the first and second shell parts may have different thicknesses (in radial direction), as e.g. shown in Fig. 7, or identical or substantially identical thicknesses. Further, the thickness of a particular shell part, in circumferential direction, is preferably constant, but may also vary, or there may be sections of different thickness.

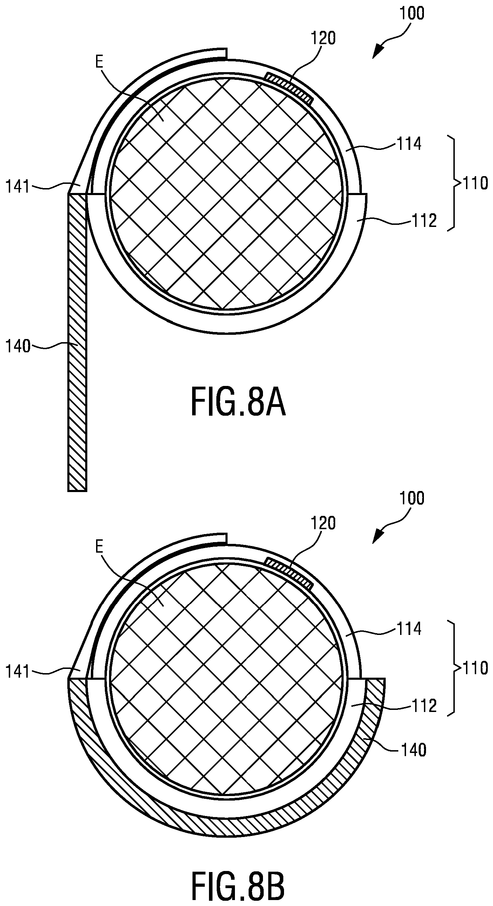

Fig. 8 shows a schematic diagram of a fourth embodiment of a cuff 100 according to the present invention, wherein Fig. 8A shows it in a deflated state and Fig. 8B shows it in an inflated state (the closing elements are not shown). In this embodiment, the actuator 140 is fixedly held, via a fixation 141, at the outer surface of the second shell part 114. The fixation 141 may e.g. be glued or fixed with Velcro fastener or otherwise fixed to the second shell part 114, while the actuator 140 (e.g. an inflatable bladder) may freely hang only at the fixation 141 as shown in Fig. 8A. The two shell parts 112, 114 can thus be separately mounted to and demounted from the body part E, wherein in the demounted state the actuator 140 is fixed only to the second shell part 114. When the cuff 100 is mounted to the body part E, the actuator will be attached (e.g. via Velcro fastener) to the outer surface of the first shell part 112 and then inflated as shown in Fig. 8B so that it pressurizes the first shell part 112 onto the body part E.

In a modification of the cuff shown in Fig. 8, the actuator 140 may comprise another fixation (not shown) at its free end that extends over the complete outer surface of the first shell part 112 and even over the adjacent part (on the right-hand side in Fig. 8B) of the outer surface of the second shell part 114 so that the actuator is not only (releasably) fixed to the outer surface of the first shell part 112 but is further (releasably) held via this additional fixation at the second shell part 114 . The fixations and the actuator may thus extend and be held over the complete (or at least a large part of the) circumference of the shell 110. In such an embodiment closing elements as shown in Figs. 3 and 5 may even be omitted since the fixations and the actuator may fulfill the purpose of holding the shell parts 112, 114 together when mounted to the body part.

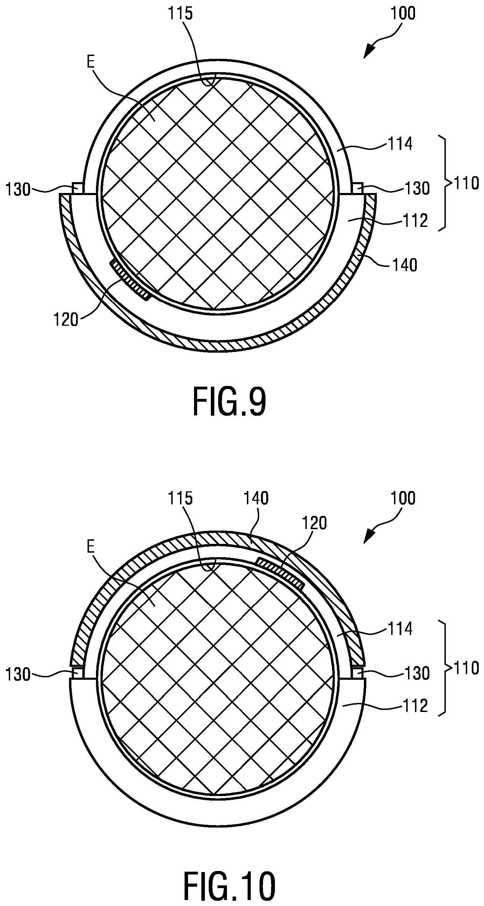

Fig. 9 shows a schematic diagram of a fifth embodiment of a cuff 100 according to the present invention. According to this embodiment the sensor unit 120 is arranged in the first shell part 112. In this case, the cuff is preferably mounted to the patient's arm such that the first shell part 112 is arranged at the inner part of the upper arm. The actuator 140 thus directly compresses the first shell part 112 and thus the area of the tissue where the brachial artery is located. Since BP and other hemodynamic parameters are measured when the brachial artery is collapsing, which signal is captured by the sensor unit 140, this arrangement of the sensor unit and the actuator 140 has advantages. Generally, in order to make the brachial artery to collapse a lot of pressure is generally to be applied so that the soft tissue is displaced, and the muscle starts contracting, which is best achieved with arrangements where the sensor unit 120 is arranged on the same side of the shell as the actuator 140 because then the external pressure exerted by the actuator 140 may generally be higher and the brachial artery collapses faster.

Fig. 10 shows a schematic diagram of a sixth embodiment of a cuff 100 according to the present invention, in which the sensor unit 120 and the actuator 140 are arranged on the same side of the cuff. Differently from the fifth embodiment shown in Fig. 9, however, in this embodiment the sensor unit 120 is arranged in the second shell part 114 and the actuator is arranged around at least part of the second shell part 114. In this case, the cuff is preferably mounted to the patient's arm such that the second shell part 112 is arranged at the inner part of the upper arm.

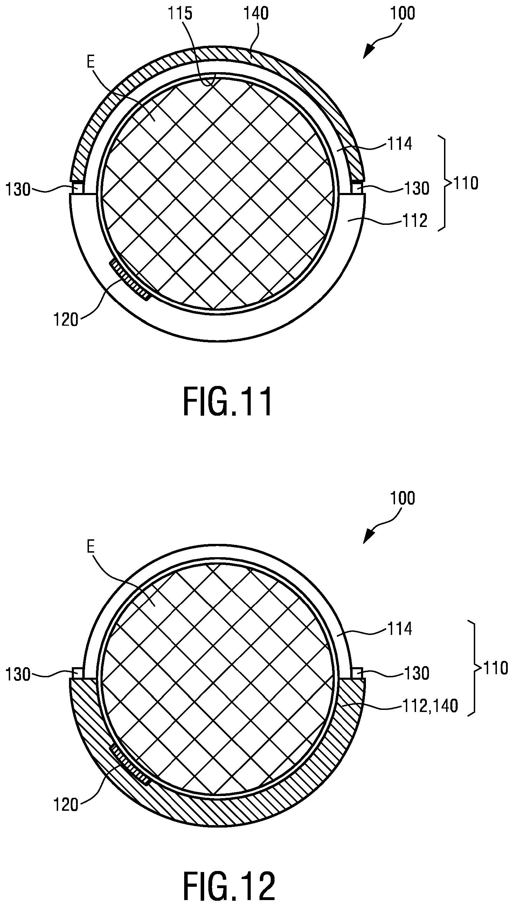

Fig. 11 shows a schematic diagram of a seventh embodiment of a cuff 100 according to the present invention, in which the sensor unit 120 is arranged in the first shell part and the actuator 140 is arranged around at least part of the second shell part 114.

Fig. 12 shows a schematic diagram of an eighth embodiment of a cuff 100 according to the present invention. This embodiment is similar to the third embodiment shown in Fig. 7, but different from this embodiment the sensor unit 120 is arranged in the first shell part 112 which directly serves as actuator 140.

In the embodiments shown in the figures the shell parts 112, 114 are configured as half shells each being configured to enclose the body part E in a circumferential area of substantially 180°. In other embodiments, however, the shell 110 may be segmented into more than two shell parts, or the two shell parts 112, 114 may be configured such that one of them encloses more than 180° (e.g. up to 270°) of the body part and the other one of them encloses less than 180° (e.g. 90° or more) of the body part, together enclosing substantially 360°.

This cuff and the system according to the present invention thus further improve the known cuff in one or more aspects, in particular with respect to one or more of signal quality, actuator controller requirements, clinical workflow, patient safety and comfort, transport and storage.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

Any reference signs in the claims should not be construed as limiting the scope.

## Claims

1. A cuff for hemodynamic monitoring, comprising:
- a shell (110) configured to enclose a subject's body part (E) during hemodynamic monitoring, the shell comprising at least a first shell part (112) and a second shell part (114) having a higher rigidity than the first shell part (112), each shell part being configured for enclosing a different circumferential portion of the body part (E);
- a sensor unit (120) arranged in or at an inner surface of at least one shell part and configured to measure hemodynamic signals;
- one or more closing elements (130, 131, 132) arranged in or at at least one shell part and configured to hold the shell parts (112, 114) together when mounted at the body part; and
- an actuator (140) configured to pressurize and/or compress at least one of the shell parts (112, 114),
wherein the first shell part (112) is compressible and/or pressurizable and the second shell part (114) is rigid or semi-rigid, and
wherein i) the first shell part (112) represents the actuator or integrates the actuator or ii) the actuator (140) is arranged on an outer surface of the first shell part (112) or the second shell part (114).

2. The cuff according to claim 1,
wherein the first shell part (112) is compressible by more than 20 % or by more than 30 %, and the second shell part (114) is not compressible or less compressible than the first shell.

3. The cuff according to claim 2,
wherein the first shell part (112) is made of a material, in particular polymer and/or foam and/or other shape memory material, that can adapt to the shape of the body part when compressed.

4. The cuff according to any one of claims 1 to 3,
further comprising a fixation (141) that is fixed at an outer surface of the first shell part or the second shell part (114) and coupled to the actuator (140), wherein the actuator is configured to be removably fixed to an outer surface of the first shell part (112) or the second shell part (114) when the cuff is mounted onto the body part.

5. The cuff according to any one of the preceding claims,
wherein the shell (110) has a conical shape and/or forms a concentrical shell without overlapping portions in circumferential direction.

6. The cuff according to any one of the preceding claims,
wherein the first shell part (112) or the second shell part (114) is configured to be arranged at an inner part of the upper arm or an outer part of the upper arm.

7. The cuff according to any one of the preceding claims,
wherein the shell parts (112, 114) are configured as half shells each being configured to enclose the body part in a circumferential area of substantially 180°.

8. The cuff according to any one of the preceding claims,
wherein the one or more closing elements comprise magnetic elements (131, 132) arranged at one or more opposing end surfaces of the shell parts (112, 114).

9. The cuff according to any one of the preceding claims,
wherein the sensor unit (120) is integrated into the inner surface or arranged onto the inner surface of the first shell part (112) or the second shell part (114).

10. The cuff according to any one of the preceding claims,
wherein the sensor unit (120) comprises:
- a sensor pad (121) filled with a fluid (122);
- a pressure transducer (123) configured to convert a fluid pressure into an electrical measurement signal; and
- a fluid connection (124) between the sensor pad and the pressure transducer.

11. The cuff according to any one of the preceding claims,
wherein the actuator (140) comprises an inflatable bladder and an air connection connected at one end to the inflatable bladder.

12. A system for hemodynamic monitoring, comprising:
- a cuff (100) as claimed in any one of the preceding claims;
- an actuator controller (210) connected to the actuator (140) of the cuff and configured to control the actuator to press the at least one of the shell parts (112, 114) of the cuff onto the body part (E); and
- a monitor (220) connected to the sensor unit and configured to obtain a measurement signal from the sensor unit.

13. The system as claimed in claim 12,
wherein the actuator controller (210) comprises an air pump (212) to inflate an inflatable bladder of the actuator through an air hose (214).

## Patentansprüche

1. Manschette zur hämodynamischen Überwachung, umfassend:
- eine Schale (110), die so ausgestaltet ist, dass sie einen Körperteil (E) eines Subjekts während einer hämodynamischen Überwachung umschließt, wobei die Schale mindestens einen ersten Schalenteil (112) und einen zweiten Schalenteil (114) umfasst, der eine höhere Steifigkeit aufweist als der erste Schalenteil (112), wobei jeder Schalenteil so ausgestaltet ist, dass er einen anderen Umfangsabschnitt des Körperteils (E) umschließt;
- eine Sensoreinheit (120), die in oder an einer Innenfläche mindestens eines Schalenteils angeordnet und so ausgestaltet ist, dass sie hämodynamische Signale misst;
- ein oder mehrere Verschlusselemente (130, 131, 132), die in oder an mindestens einem Schalenteil angeordnet und so ausgestaltet sind, dass sie die Schalenteile (112, 114) zusammenhalten, wenn diese an dem Körperteil angebracht werden; und
- eine Betätigungsvorrichtung (140), die so ausgestaltet ist, dass sie mindestens einen der Schalenteile (112, 114) mit Druck beaufschlagt und/oder komprimiert,
wobei der erste Schalenteil (112) komprimierbar und/oder mit Druck beaufschlagbar ist, und der zweite Schalenteil (114) starr oder halbstarr ist, und
wobei i) der erste Schalenteil (112) die Betätigungsvorrichtung darstellt oder die Betätigungsvorrichtung integriert, oder ii) die Betätigungsvorrichtung (140) an einer Außenfläche des ersten Schalenteils (112) oder des zweiten Schalenteils (114) angeordnet ist.

2. Manschette nach Anspruch 1,
wobei der erste Schalenteil (112) um mehr als 20% oder um mehr als 30% komprimierbar ist, und der zweite Schalenteil (114) nicht komprimierbar ist oder weniger komprimierbar als die erste Schale.

3. Manschette nach Anspruch 2,
wobei der erste Schalenteil (112) aus einem Material, insbesondere Polymer und/oder Schaumstoff und/oder anderem Formgedächtnismaterial besteht, das sich, wenn es komprimiert wird, an die Form des Körperteils anpassen kann.

4. Manschette nach einem der Ansprüche 1 bis 3,
die weiter eine Befestigung (141) umfasst, die an einer Außenfläche des ersten Schalenteils oder des zweiten Schalenteils (114) befestigt und mit der Betätigungsvorrichtung (140) gekoppelt ist, wobei die Betätigungsvorrichtung so ausgestaltet ist, dass sie abnehmbar an einer Außenfläche des ersten Schalenteils (112) oder des zweiten Schalenteils (114) befestigt werden kann, wenn die Manschette an dem Körperteil angebracht wird.

5. Manschette nach einem der vorstehenden Ansprüche,
wobei die Schale (110) eine konische Form aufweist und/oder eine konzentrische Schale ohne überlappende Abschnitte in Umfangsrichtung bildet.

6. Manschette nach einem der vorstehenden Ansprüche,
wobei der erste Schalenteil (112) oder der zweite Schalenteil (114) so ausgestaltet ist, dass er an einem inneren Teil des Oberarms oder einem äußeren Teil des Oberarms angeordnet werden kann.

7. Manschette nach einem der vorstehenden Ansprüche,
wobei die Schalenteile (112, 114) als Halbschalen ausgestaltet sind, die jeweils so ausgestaltet sind, dass sie den Körperteil in einem Umfangsbereich von im Wesentlichen 180° umschließen.

8. Manschette nach einem der vorstehenden Ansprüche,
wobei das eine oder die mehreren Verschlusselemente Magnetelemente (131, 132) umfassen, die an einer oder mehreren gegenüberliegenden Endflächen der Schalenteile (112, 114) angeordnet sind.

9. Manschette nach einem der vorstehenden Ansprüche,
wobei die Sensoreinheit (120) in die Innenfläche des ersten Schalenteils (112) oder des zweiten Schalenteils (114) integriert oder auf dieser Innenfläche angeordnet ist.

10. Manschette nach einem der vorstehenden Ansprüche,
wobei die Sensoreinheit (120) umfasst:
- ein Sensorpad (121), das mit einem Fluid (122) gefüllt ist;
- ein Druckaufnehmer (123), der so ausgestaltet ist, dass er einen Fluiddruck in ein elektrisches Messsignal umwandelt; und
- eine Fluidverbindung (124) zwischen dem Sensorpad und dem Druckaufnehmer.

11. Manschette nach einem der vorstehenden Ansprüche,
wobei die Betätigungsvorrichtung (140) eine aufblasbare Blase und einen Luftanschluss umfasst, der an einem Ende mit der aufblasbaren Blase verbunden ist.

12. System zur hämodynamischen Überwachung, umfassend:
- eine Manschette (100) nach einem der vorstehenden Ansprüche;
- eine Betätigungsvorrichtungs-Steuereinheit (210), die mit der Betätigungsvorrichtung (140) der Manschette verbunden und so ausgestaltet ist, dass sie die Betätigungsvorrichtung so steuert, dass sie den mindestens einen der Schalenteile (112, 114) der Manschette an den Körperteil (E) drückt; und
- einen Monitor (220), der mit der Sensoreinheit verbunden und so ausgestaltet ist, dass er ein Messsignal von der Sensoreinheit erhält.

13. System nach Anspruch 12,
wobei die Betätigungsvorrichtungs-Steuereinheit (210) eine Luftpumpe (212) umfasst, um eine aufblasbare Blase der Betätigungsvorrichtung durch einen Luftschlauch (214) aufzublasen.

## Revendications

1. Brassard pour la surveillance hémodynamique, comprenant :
- une enveloppe (110) configurée pour envelopper une partie du corps d'un sujet (E) pendant la surveillance hémodynamique, l'enveloppe comprenant au moins une première partie d'enveloppe (112) et une seconde partie d'enveloppe (114) présentant une rigidité supérieure à celle de la première partie d'enveloppe (112), chaque partie d'enveloppe étant configurée pour envelopper une portion circonférentielle différente de la partie du corps (E) ;
- une unité de capteur (120) disposée dans ou au niveau d'une surface intérieure d'au moins une partie d'enveloppe et configurée pour mesurer les signaux hémodynamiques ;
- un ou plusieurs éléments de fermeture (130, 131, 132) disposés dans ou au niveau d'au moins une partie d'enveloppe et configurés pour maintenir les parties d'enveloppe (112, 114) ensemble lorsqu'elles sont montées sur la partie du corps ; et
- un actionneur (140) configuré pour pressuriser et/ou comprimer au moins une des parties d'enveloppe (112, 114),
dans lequel la première partie d'enveloppe (112) est compressible et/ou pressurisable et la seconde partie d'enveloppe (114) est rigide ou semi-rigide, et
dans lequel i) la première partie d'enveloppe (112) représente l'actionneur ou intègre l'actionneur ou ii) l'actionneur (140) est disposé sur une surface extérieure de la première partie d'enveloppe (112) ou de la seconde partie d'enveloppe (114).

2. Brassard selon la revendication 1,
dans lequel la première partie d'enveloppe (112) est compressible de plus de 20 % ou de plus de 30 %, et la seconde partie d'enveloppe (114) n'est pas compressible ou est moins compressible que la première enveloppe.

3. Brassard selon la revendication 2,
dans lequel la première partie d'enveloppe (112) est constituée d'un matériau, notamment d'un polymère et/ou d'une mousse et/ou d'un autre matériau à mémoire de forme, qui peut s'adapter à la forme de la partie du corps lorsqu'il est comprimé.

4. Brassard selon l'une quelconque des revendications 1 à 3,
comprenant en outre une fixation (141) qui est fixée à une surface extérieure de la première partie d'enveloppe ou de la seconde partie d'enveloppe (114) et couplée à l'actionneur (140), dans lequel l'actionneur est configuré pour être fixé de manière amovible à une surface extérieure de la première partie d'enveloppe (112) ou de la seconde partie d'enveloppe (114) lorsque le brassard est monté sur la partie du corps.

5. Brassard selon l'une quelconque des revendications précédentes,
dans laquelle l'enveloppe (110) présente une forme conique et/ou forme une enveloppe concentrique sans chevauchement de parties dans la direction circonférentielle.

6. Brassard selon l'une quelconque des revendications précédentes,
dans lequel la première partie d'enveloppe (112) ou la seconde partie d'enveloppe (114) est configurée pour être disposée dans une partie intérieure de la partie supérieure du bras ou dans une partie extérieure de la partie supérieure du bras.

7. Brassard selon l'une quelconque des revendications précédentes,
dans lequel les parties d'enveloppe (112, 114) sont configurées comme des demi-enveloppes, chacune étant configurée pour envelopper la partie du corps dans une zone circonférentielle sensiblement de 180°.

8. Brassard selon l'une quelconque des revendications précédentes,
dans lequel les un ou plusieurs éléments de fermeture comprennent des éléments magnétiques (131, 132) disposés au niveau d'une ou de plusieurs surfaces d'extrémité opposées des parties d'enveloppe (112, 114).

9. Brassard selon l'une quelconque des revendications précédentes,
dans lequel l'unité de capteur (120) est intégrée dans la surface intérieure ou disposée sur la surface intérieure de la première partie d'enveloppe (112) ou de la seconde partie d'enveloppe (114).

10. Brassard selon l'une quelconque des revendications précédentes,
dans lequel l'unité de capteur (120) comprend :
- un coussinet de capteur (121) rempli d'un fluide (122) ;
- un transducteur de pression (123) configuré pour convertir une pression de fluide en un signal de mesure électrique ; et
- une connexion de fluide (124) entre le coussinet de capteur et le transducteur de pression.

11. Brassard selon l'une quelconque des revendications précédentes,
dans lequel l'actionneur (140) comprend une vessie gonflable et une connexion d'air reliée à une extrémité à la vessie gonflable.

12. Système de surveillance hémodynamique, comprenant :
- un brassard (100) selon l'une quelconque des revendications précédentes ;
- un dispositif de commande d'actionneur (210) connecté à l'actionneur (140) du brassard et configuré pour commander l'actionneur afin qu'il presse l'au moins une des parties d'enveloppe (112, 114) du brassard sur la partie du corps (E) ; et
- un moniteur (220) connecté à l'unité de capteur et configuré pour obtenir un signal de mesure de l'unité de capteur.

13. Système selon la revendication 12,
dans lequel le dispositif de commande d'actionneur (210) comprend une pompe à air (212) pour gonfler une vessie gonflable de l'actionneur à travers un tuyau d'air (214).
